# EUROPEAN PATENT SPECIFICATION

(11) **EP 3 514 144 B1**
(45) Date of publication and mention of the grant of the patent: **17.08.2022**
(21) Application number: 17851014.5
(22) Date of filing: 15.09.2017
(51) Int. Cl.: C07D 213/75

(54) **OPTIMIZED PRODUCTION METHOD FOR PEST CONTROL AGENT**
OPTIMIERTES HERSTELLUNGSVERFAHREN FÜR SCHÄDLINGSBEKÄMPFUNGSMITTEL
PROCÉDÉ DE PRODUCTION OPTIMISÉ POUR AGENT DE LUTTE ANTIPARASITAIRE

(30) Priority: 16.09.2016 JP 2016181235
(43) Date of publication of application: 24.07.2019
(60) Divisional application: 22181005.4
(73) Proprietor: MMAG Co., Ltd., Tokyo 103-0027 (JP)
(72) Inventor: KITSUDA Shigeki, Yokohama-shi Kanagawa 222-8567 (JP); NAKANISHI Nozomu, Yokohama-shi Kanagawa 222-8567 (JP); SUMI Shinjiro, Yokohama-shi Kanagawa 222-8567 (JP)
(74) Representative: J A Kemp LLP
(86) International application number: PCT/JP2017/033461
(87) International publication number: WO 2018/052115

(56) References cited:
- WO-A1-2012/029672
- WO-A1-2015/086790
- WO-A1-2015/137216
- WO-A1-2016/005276
- WO-A2-2013/129692

## Description

### [Technical Field]

The present invention relates to an optimized method for producing a pest control agent having a 2-acyliminopyridine structure.

### [Background Art]

2-Acyliminopyridine derivatives such as N-[1-((6-chloropyridin-3-yl)methyl)pyridin-2(1H)-yli dene]-2,2,2-trifluoroacetamide represented by formula (I) to be described later are compounds useful as pest control agents (Patent Literature 1).

Related art documents (Patent Literatures 1 to 4) are known as methods for producing a pest control agent having a 2-acyliminopyridine structure.

Patent Literatures 1 and 2 describe a method for producing the compound represented by formula (I) via an acylation reaction using a trifluoroacetic acid ester. However, the method uses a large amount of trifluoroacetic acid ester reagent, and the yield is low. Although Patent Literature 4 also describes a method for producing the compound mentioned above using a trifluoroacetic acid ester, but the yield is low and the reaction requires a long period of time. For these reasons, the production methods described in these literatures are not suitable for industrial production.

In addition, Patent Literature 3 also describes a specific method for producing the compound represented by formula (I). However, the reagent used in the acylation step is limited to trifluoroacetic acid, and neither description nor suggestion is provided for the method which uses a trifluoroacetic acid ester as disclosed in Patent Literatures 1, 2, and 4.

In summary, to date, there has been no method for producing the compound represented by formula (I) which is suitable for industrial production and which makes it possible to obtain the compound in a high yield and in a short period of time using a trifluoroacetic acid ester.

### [Citation List]

### [Patent Literature]

[PTL 1] International Publication No. WO 2012/029672
[PTL 2] International Publication No. WO 2013/031671
[PTL 3] International Publication No. WO 2015/137216
[PTL 4] International Publication No. WO 2016/005276
[PTL 5] Japanese Unexamined Patent Application Publication No. 2003-321441

### [Non Patent Literature]

[NPL 1] Russian Journal of organic chem 48 (10) P 1297-1301, 2012
[NPL 2] Tetrahedron 59, P 9019-9029, 2003
[NPL 3] Bioorg. Med. Chem 10, P 3175-3185, 2002

### [Summary of Invention]

### [Technical Problem]

An object of the present invention is to provide a method for producing N-[1-((6-chloropyridin-3-yl)methyl)pyridin-2(1H)-yli dene]-2,2,2-trifluoroacetamide represented by formula (I) to be described later in a high yield and in a short period of time, and further to provide a method for stably, inexpensively, and industrially producing the compound in an amount required as a pest control agent.

### [Solution to Problem]

The present inventors have found that, in a production method for obtaining a compound represented by the following formula (I) by using a compound represented by formula (A) as a starting substance, and a compound represented by formula (B) as an intermediate, the use of a trifluoroacetic acid ester and a metal base makes it possible to produce a desired compound industrially and economically efficiently while consuming the reagent in a small amount. As a result, the present invention has been completed.

Note that although Patent Literature 5 and Non Patent Literatures 1 to 3 describe methods for introducing a trifluoroacetyl group to an amino group using a trifluoroacetic acid ester and a metal base, the reaction substrate and product are compounds completely different from the compound represented by formula (I) . Thus, the present invention is not suggested.

Specifically, the present invention provides a method for producing the compound represented by the following formula (I) shown below.
<1> A method for producing a compound represented by the following formula (I) 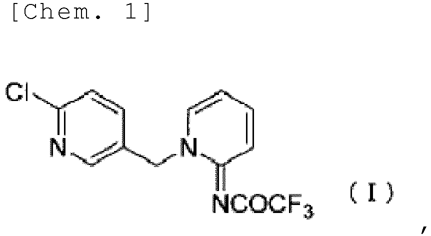 the method comprising, as shown in the following reaction formula: 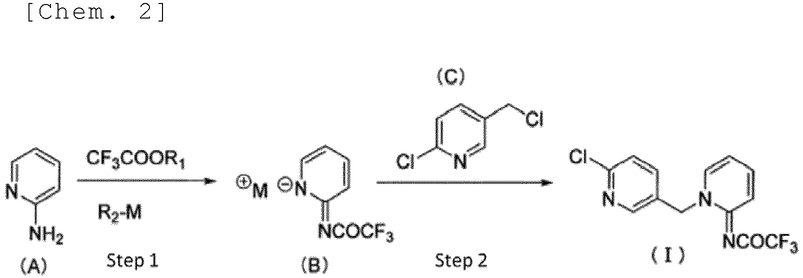 [where R₁ represents an ethyl group, R₂ represents a methyloxy group, and M represents a sodium atom,
   step 1 of producing a compound represented by formula (B) by acylating an amino group at position 2 of a compound represented by formula (A) using a trifluoroacetic acid ester and a metal base; and
   step 2 of alkylating a nitrogen atom at position 1 of the compound represented by formula (B) using a compound represented by formula (C), and wherein
   the acylation reaction using the trifluoroacetic acid ester is performed in a solvent which consists of N,N-dimethylformamide and methanol.
<2> The production method according to <1>, wherein the step 2 includes alkylating the nitrogen atom at position 1 of the compound represented by formula (B) using the compound represented by formula (C) in a solvent containing N,N-dimethylformamide.
<3> The production method according to any one of <1> to <2>, wherein in the step 1, an amount of the metal base used is 0.95 to 1.1 equivalents relative to the compound represented by formula (A), and an amount of the trifluoroacetic acid ester used is 1.0 to 1.5 equivalents relative to the compound represented by formula (A).
<4> A method for producing a compound represented by formula (I), comprising producing the compound represented by formula (I) in a one-pot manner from a compound represented by formula (A) by adding a trifluoroacetic acid ester, a metal base, and a compound represented by formula (C) in the same reaction vessel as shown in the following reaction formula: 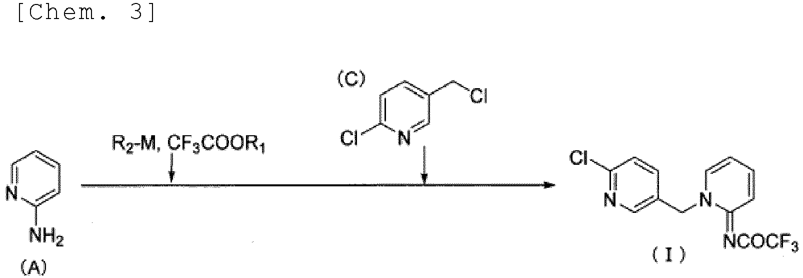 [where R₁ represents an ethyl group, R₂ represents a methyloxy group, and M represents a sodium atom, and wherein
   an acylation reaction using the trifluoroacetic acid ester is performed in a solvent which consists of N,N-dimethylformamide and methanol.
<5> The production method according to <4> wherein
   an alkylation reaction using the compound represented by formula (C) is performed in a solvent containing N,N-dimethylformamide.

### [Advantageous Effects of Invention]

The present invention makes it possible to produce the compound represented by formula (I), which is useful as a pest control agent, in a short period of time and in a high yield. In addition, if necessary, the compound can also be produced in the same reaction vessel. In other words, the present invention makes it possible to produce the compound advantageously from an industrial and economical point of view.

### [Description of Embodiments]

In the present specification, a "salt" refers to, for example, an inorganic acid salt and an organic acid salt. Examples of the inorganic acid salt include hydrochlorides, sulfates, nitrates, sodium salts, and potassium salts. Examples of the organic acid salt include trifluoroacetates, difluoroacetates, and dichloroacetates.

### [Production Method]

The present invention will be further explained in detail according to the following scheme.

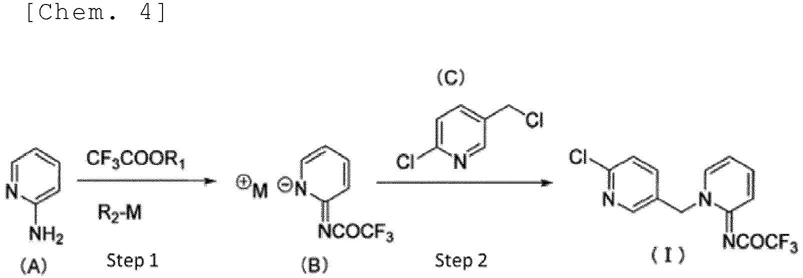

### [1] Production of the compound represented by formula (B) from the compound represented by formula (A) (step 1)

Production of the compound represented by formula (B) from the compound represented by formula (A) by use of a trifluoroacetic acid ester (CF₃COOR₁) is as follows. Specifically, the production can be performed by acylation of the compound represented by formula (A) in a solvent which does not affect the reaction and in the presence of a metal base (R₂-M), wherein said solvent consists of N,N-dimethylformamide and methanol.

The usable metal base (R₂-M) is sodium methoxide. The reaction is carried out in the presence of a base, and the amount of the base used is, for example, 0.5 to 2.0 equivalents, preferably 0.9 to 1.2 equivalents, and more preferably 0.95 to 1.1 equivalents relative to the compound represented by formula (A) (2-aminopyridine). The metal base can also be used by being dissolved in alcohol-based solvents such as methanol.

The trifluoroacetic acid ester (CF₃COOR₁) is ethyl trifluoroacetate. The equivalent amount of the trifluoroacetic acid ester is preferably 0.9 to 2.0 equivalents and more preferably 1.0 to 1.5 equivalents relative to the compound represented by formula (A) (2-aminopyridine).

The reaction temperature is preferably in a range from -80°C to 80°C, more preferably in a range from 5°C to 55°C, and further preferably between 25°C and 45°C. The reaction time is preferably in a range from 0.1 hours to 7 days and more preferably between 1 hour and 7 hours. In addition, after completion of the reaction, the alcohol (R₁-OH) as a byproduct and the alcohol used as a solvent are preferably distilled off under reduced pressure.

### [2] Production of the compound represented by formula (I) from the compound represented by formula (B) (step 2)

Production of the compound represented by formula (I) from the compound represented by formula (B) is as follows. Specifically, the production can be performed by alkylation reaction of the compound represented by formula (B) with the compound represented by formula (C) without a solvent or in a solvent.

Examples of usable solvents include hydrocarbon-based solvents, ester-based solvents, ether-based solvents, aprotic polar organic solvents, halogen-containing solvents, hydrocarbon-based solvents, ketone-based solvents, alcohol-based solvents, and water as well as mixture solvents containing at least one of these solvents. Specifically, examples of the hydrocarbon-based solvents include toluene, xylene, ethylbenzene, normal hexane, and cyclohexane; examples of the ester-based solvents include methyl acetate, ethyl acetate, and butyl acetate; examples of the ether-based solvents include diethyl ether, diisopropyl ether, tetrahydrofuran, dioxane, dimethoxyethane, and tert-butyl methyl ether; examples of the aprotic polar organic solvents include N,N-dimethylformamide, dimethyl sulfoxide, N,N-dimethylacetamide, N-methyl-2-pyrrolidone, 1,3-dimethyl-2-imidazolidinone, and acetonitrile; examples of the halogen-containing solvents include dichloromethane and chloroform; examples of the hydrocarbon-based solvents include cyclohexane; examples of the ketone-based solvents include acetone and methyl ethyl ketone; and examples of the alcohol-based solvents include methanol, ethanol, 2-propanol, and normal butanol.

Preferable examples of the solvents usable in the above-described alkylation reaction include aprotic polar organic solvents, more preferably one or two or more solvents selected from the group consisting of N,N-dimethylformamide, dimethyl sulfoxide, N,N-dimethylacetamide, N-methyl-2-pyrrolidone, 1,3-dimethyl-2-imidazolidinone, and acetonitrile, and particularly preferably N,N-dimethylformamide.

The reaction temperature is preferably in a range from 20°C to 100°C, more preferably from 40°C to 90°C, and further preferably from 60°C to 70°C. The reaction time is preferably in a range from 0.1 hours to 3 days and more preferably in a range from 1 hour and 1 day.

A particularly preferable form of the present invention is the form satisfying all of the following in the scheme described above.

A method for producing the compound represented by formula (I) from the compound represented by formula (A) in the same reaction vessel, comprising: step 1 of using ethyl trifluoroacetate as an acylating agent, sodium methoxide as a metal base (R₂M), and a solvent consisting of N,N-dimethylformamide and methanol as a solvent; and further step 2 of using N,N-dimethylformamide as a solvent, wherein the amount of the metal base used in step 1 is 0.95 to 1.1 equivalents, the amount of the ethyl trifluoroacetate used is 1.0 to 1.5 equivalents, the reaction temperature in step 1 here is between 25°C to 45°C, and the reaction temperature in step 2 is between 60°C to 70°C.

The compound represented by formula (B) shown in the above scheme in the present invention may be used for the subsequent step as it is without post treatment or isolation. As shown in the following scheme (following reaction formula), it is preferable to produce the compound represented by formula (I) from the compound represented by formula (A) in the same reaction vessel in a one-pot manner.

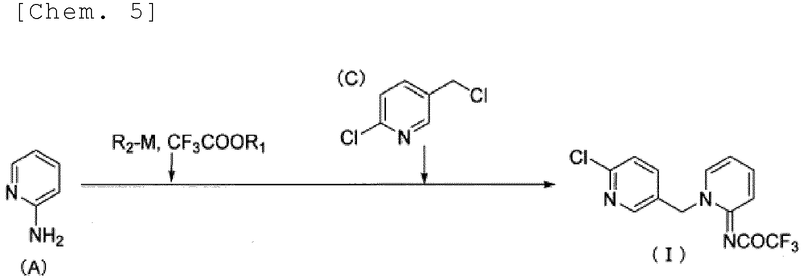

The definitions of R₁, R₂, and M in the above reaction formula are as described above. In this case, it is preferable to remove the solvent by e.g. distillation before the addition of the compound represented by formula (C) as needed.

### [Examples]

Specific examples of the present invention are shown below, but the present invention is not limited these.

### (Example 1)

Added were 31.24 g (0.22 mol) of ethyl trifluoroacetate, 18.82 g (0.20 mol) of 2-aminopyridine, and 16 g of N,N-dimethylformamide (DMF) in this order, and after dissolution, 38.57 g (0.20 mol) of sodium methoxide (28.0% methanol solution) was added dropwise thereto at room temperature. After stirring at 25°C for 1 hour, methanol and ethanol were distilled off under reduced pressure. A solution prepared by dissolving 32.70 g (0.20 mol) of 2-chloro-5-chloromethyl pyridine in 18.8 g of DMF was added thereto, followed by stirring at 60°C for 3 hours and 15 minutes. Thereafter, 110 ml of water was added, and after stirring at room temperature for 3 hours, the precipitate was collected by filtration. After pushing and washing twice with 40 ml of water, vacuum drying at 70°C overnight was performed to obtain 59.81 g of the desired product (yield 94.7% and purity 98.6%).

### (Examples 2 to 4 and Comparative Example 1)

In Examples 2 to 4, the desired products having the yields and purities shown in Table 1 were obtained by reaction in which the equivalent amount of the ethyl trifluoroacetate in step 1 of Example 1 relative to the 2-aminopyridine was changed as shown in Table 1. In Comparative Example 1, reaction was performed by changing the equivalent amount of the ethyl trifluoroacetate relative to 1 equivalent of the 2-aminopyridine in step 1 of Example 1 as shown in Table 1. Consequently, the desired product was obtained, but the results were such that the yield was inferior to the yields of Examples 1 to 4, as shown in Table 1.

**[Table 1]**

| | *Equivalent Amount of Ethyl Trifluoroacetate Relative to 2-Aminopyridine | Yield | Purity |
|---|---|---|---|
| Comparative Example 1 | 0.90 equivalents | 76.5% | 98.5% |
| Example 2 | 1.00 equivalent | 88.2% | 100.0% |
| Example 1 | 1.10 equivalents | 94.7% | 98.6% |
| Example 3 | 1.20 equivalents | 92.1% | 98.0% |
| Example 4 | 1.50 equivalents | 88.5% | 99.3% |

### (Examples 5 to 7 and Comparative Examples 2 and 3)

In Examples 5 to 7, the desired products having the yields and purities shown in Table 2 were obtained by reaction in which the equivalent amount of the sodium methoxide relative to 1 equivalent of the 2-aminopyridine in step 1 of Example 1 was changed as shown in Table 2. In Comparative Examples 2 and 3, reaction was performed by changing the equivalent amount of the sodium methoxide relative to 1 equivalent of the 2-aminopyridine in step 1 of Example 1 as shown in Table 2. Consequently, the desired products were obtained, but the results were such that the yields were inferior to the yields of Examples 1 and 5 to 7, as shown in Table 2.

**[Table 2]**

| | Equivalent Amount of Sodium Methoxide Relative to 2-Aminopyridine | Yield | Purity |
|---|---|---|---|
| Comparative Example 2 | 0.90 equivalents | 75.5% | 99.3% |
| Example 5 | 0.95 equivalents | 85.3% | 99.0% |
| Example 1 | 1.00 equivalent | 94.7% | 98.6% |
| Example 6 | 1.05 equivalents | 92.1% | 99.3% |
| Example 7 | 1.10 equivalents | 88.4% | 99.5% |
| Comparative Example 3 | 1.20 equivalents | 80.1% | 98.9% |

### (Examples 8 to 10)

Added were 31.24 g (0.22 mol) of ethyl trifluoroacetate, 18.82 g (0.20 mol) of 2-aminopyridine, and 16 g of DMF in this order, and after dissolution, 38.57 g (0.20 mol) of sodium methoxide (28.0% methanol solution) was added dropwise thereto at room temperature. After stirring at each temperature shown in Table 3 (5°C, 25°C, 45°C, or 55°C), methanol and ethanol were distilled off under reduced pressure. A solution prepared by dissolving 32.70 g (0.20 mol) of 2-chloro-5-chloromethyl pyridine in 18.8 g of DMF was added thereto, followed by stirring at 60°C. Thereafter, 110 ml of water was added, and after stirring at room temperature for 3 hours, the precipitate was collected by filtration. After pushing and washing twice with 40 ml of water, vacuum drying at 70°C overnight was performed to obtain as a result the desired products having the yields and purities shown in Table 3.

**[Table 3]**

| | Reaction Temperature in Step 1 | Yield | Purity |
|---|---|---|---|
| Example 8 | 5°C | 87.3% | 98.9% |
| Example 1 | 25°C | 94.7% | 98.6% |
| Example 9 | 45°C | 90.4% | 98.9% |
| Example 10 | 55°C | 86.2% | 99.2% |

### (Examples 11 and 12 and Comparative Example 4)

Added were 31.24 g (0.22 mol) of ethyl trifluoroacetate, 18.82 g (0.20 mol) of 2-aminopyridine, and 16 g of DMF in this order, and after dissolution, 38.57 g (0.20 mol) of sodium methoxide (28.0% methanol solution) was added dropwise thereto at room temperature. After stirring at 25°C for 1 hour, methanol and ethanol were distilled off under reduced pressure. A solution prepared by dissolving 32.70 g (0.20 mol) of 2-chloro-5-chloromethyl pyridine in 18.8 g of DMF was added thereto, followed by stirring at each temperature shown in Table 4 (50°C or 70°C) for 1 to 7 hours. Thereafter, 110 ml of water was added, and after stirring at room temperature for 3 hours, the precipitate was collected by filtration. After pushing and washing twice with 40 ml of water, vacuum drying at 70°C overnight was performed to obtain as a result the desired products having the yields and purities shown in Table 4. In addition, in Comparative Example 4, reaction was performed by changing the above temperature in step 2 to 80°C, but the but results were such that the yield was inferior to the yields of Examples 1, 11, and 12, as shown in Table 4.

**[Table 4]**

| | Reaction Temperature in Step 2 | Yield | Purity |
|---|---|---|---|
| Example 11 | 50°C | 89.7% | 99.9% |
| Example 1 | 60°C | 94.7% | 98.6% |
| Example 12 | 70°C | 92.3% | 98.3% |
| Comparative Example 4 | 80°C | 83.2% | 99.2% |

### (Comparative Example 5)

Added were 14.09 g (0.11 mol) of methyl trifluoroacetate, 9.41 g (0.10 mol) of 2-aminopyridine, and 13 g of DMF in this order, and after dissolution, 19.29 g (0.10 mol) of sodium methoxide (28.0% methanol solution) was added dropwise thereto at room temperature. After stirring at 25°C for 40 minutes, ethanol was distilled off under reduced pressure. A solution prepared by dissolving 16.17 g (0.10 mol) of 2-chloro-5-chloromethyl pyridine in 9.4 g of DMF was added thereto, followed by stirring at 60°C for 2 hours and 20 minutes. Thereafter, 60 ml of water and 6 ml of methanol were added, and after stirring at room temperature for 1 hour, the precipitate was collected by filtration. After pushing and washing twice with 30 ml of water and twice with 20 ml of 60 v/v% aqueous solution of methanol, vacuum drying at 70°C for 8 hours was performed to obtain as a result 28.85 g of the desired product (yield 91.4% and purity 98.6%).

### (Example 13)

Added were 31.24 g (0.22 mol) of ethyl trifluoroacetate, 18.82 g (0.20 mol) of 2-aminopyridine, and 16 g of DMF in this order, and after dissolution, 68.05 g (0.20 mol) of sodium methoxide (20.0% methanol solution) was added dropwise thereto at room temperature. After stirring at room temperature for 1 hour, ethanol was distilled off under reduced pressure. A solution prepared by dissolving 32.70 g (0.20 mol) of 2-chloro-5-chloromethyl pyridine in 18.8 g of DMF was added thereto, followed by stirring at 60°C for 4 hours. Thereafter, 110 ml of water was added, and after stirring at room temperature for 2.5 hours, the precipitate was collected by filtration. After pushing and washing twice with 40 ml of water, vacuum drying at 70°C overnight was performed to obtain as a result 58.56 g of the desired product (yield 92.7% and purity 99.7%).

### (Comparative Example 6)

Added were 15.62 g (0.11 mol) of ethyl trifluoroacetate, 9.41 g (0.20 mol) of 2-aminopyridine, and 9 g of DMF in this order, and 3.86 g (0.10 mol) of sodium hydride (purity 62.2%) dissolved at room temperature was added for 6 minutes while cooling the outer side of the vessel with tap water. After stirring at room temperature for 1 hour, methanol and ethanol were distilled off under reduced pressure. Added thereto were 1.42 g (0.01 mol) of ethyl trifluoroacetate and 309 mg (0.008 mol) of sodium hydride (purity 62.2%), followed by stirring for 30 minutes. After that, a solution prepared by dissolving 16.17 g (0.092 mol) of 2-chloro-5-chloromethyl pyridine in 10 ml of DMF was added thereto, followed by stirring at 60°C for 3 hours. Thereafter, a mixture liquid of 6 ml of methanol and 60 ml of water was added, and after stirring at room temperature for 30 minutes, the precipitate was collected by filtration. The precipitate was pushed and washed twice with 30 ml of water and twice with 20 ml of 60 v/v% aqueous solution of methanol, and spread in a Petri dish and dried in the draft for 7 days to obtain as a result 29.03 g of the desired product (yield 82.0% and purity 94.7%) .

### (Comparative Example 7)

Added were 15.62 g (0.11 mol) of ethyl trifluoroacetate, 9.41 g (0.20 mol) of 2-aminopyridine, and 9 g of DMF in this order, and 11.27 g (0.10 mol) of potassium tert-butoxide (purity 99.6%) was added for 4 minutes under ice cooling. After stirring at room temperature for 1 hour and 30 minutes, methanol and tert-butanol were distilled off under reduced pressure. A solution prepared by dissolving 16.17 g (0.092 mol) of 2-chloro-5-chloromethyl pyridine in 10 ml of DMF was added thereto, followed by stirring at 60°C for 4 hours. Thereafter, a mixture liquid of 6 ml of methanol and 60 ml of water was added, and after stirring at room temperature for 30 minutes, the precipitate was collected by filtration. The precipitate was pushed and washed twice with 30 ml of water and twice with 20 ml of 60 v/v% aqueous solution of methanol, followed by vacuum drying at 0°C overnight to obtain as a result 29.03 g of the desired product (yield 92.0% and purity 99.3%).

### (Comparative Example 8)

Added were 31.24 g (0.22 mol) of ethyl trifluoroacetate and 18.82 g (0.20 mol) of 2-aminopyridine in this order, and after dissolution at 45°C, the temperature was returned to room temperature and 38.59 g (0.20 mol) of sodium methoxide (28.0% methanol solution) was added dropwise thereto. After stirring at 25°C for 30 minutes, methanol and ethanol were distilled off under reduced pressure. A solution prepared by dissolving 32.35 g (0.20 mol) of 2-chloro-5-chloromethyl pyridine in 35 ml of dimethyl sulfoxide was added thereto, followed by stirring at 60°C for 2 hours. Thereafter, 160 ml of water was added, and after stirring at room temperature for 3 hours, the precipitate was collected by filtration. The precipitate was pushed and washed twice with 40 ml of water, and spread in a Petri dish and dried in the draft overnight to obtain as a result 57.77 g of the desired product (yield 91.7% and purity 99.3%) .

### (Comparative Example 9)

Added were 15.62 g (0.22 mol) of ethyl trifluoroacetate, 9.41 g (0.10 mol) of 2-aminopyridine, and 9.4 g of N-methyl-2-pyrrolidone in this order, and after dissolution, 19.02 g (0.10 mol) of sodium methoxide (28.4% methanol solution) was added dropwise thereto at room temperature. After stirring at 25°C for 30 minutes, methanol and ethanol were distilled off under reduced pressure. A solution prepared by dissolving 16.17 g (net 0.10 mol) of 2-chloro-5-chloromethyl pyridine (purity 99.07%) in 9.4 g of N-methyl-2-pyrrolidone was added thereto, followed by stirring at 60°C for 3 hours and 15 minutes. Thereafter, 60 ml of water and 6 ml of methanol were added, and after stirring at room temperature for 2 hours and 30 minutes, the precipitate was collected by filtration. The precipitate was pushed and washed twice with 30 ml of water and twice with 20 ml of 60 v/v% aqueous solution of methanol, followed by vacuum drying at 70°C overnight to obtain as a result 27.55 g of the desired product (yield 87.3% and purity 99.8%).

### (Comparative Example 10)

Added were 15.62 g (0.22 mol) of ethyl trifluoroacetate, 9.41 g (0.10 mol) of 2-aminopyridine, and 9.4 g of N,N-dimethylacetamide in this order, and after dissolution, 19.02 g (0.10 mol) of sodium methoxide (28.4% methanol solution) was added dropwise thereto at room temperature. After stirring at 25°C for 30 minutes, methanol and ethanol were distilled off under reduced pressure. A solution prepared by dissolving 16.17 g (net 0.10 mol) of 2-chloro-5-chloromethyl pyridine (purity 99.07%) in 9.4 g of N-methyl-2-pyrrolidone was added thereto, followed by stirring at 60°C for 2 hours and 40 minutes. Thereafter, 60 ml of water and 6 ml of methanol were added, and after stirring at room temperature for 1 hour, the precipitate was collected by filtration. The precipitate was pushed and washed twice with 30 ml of water and twice with 20 ml of 60 v/v% aqueous solution of methanol, followed by vacuum drying at 70°C overnight to obtain as a result 28.31 g of the desired product (yield 89.7% and purity 99.9%).

### (Comparative Example 11)

In 4.2 g of DMF, 4.72 g (0.050 mol) of 2-aminopyridine was dissolved, and after the addition of 7.14 ml (0.060 mol) of ethyl trifluoroacetate, 2.99 g (0.054 mol) of sodium methoxide powder was added under ice cooling. The mixture was stirred at room temperature for 1 hour and then ice cooled. The mixture was added with 0.60 g (0.032 mol) of sodium methoxide and 1.4 ml (0.012 mol) of ethyl trifluoroacetate, and stirred at room temperature for 30 minutes, followed by concentration of methanol under reduced pressure. A solution prepared by dissolving 8.13 g (0.050 mol) of 2-chloro-5-chloromethyl pyridine in 10 ml of DMF was added thereto, followed by stirring at 60°C for 4 hours. Thereafter, 28 ml of water was added, and after stirring at room temperature for 1 hour, the precipitate was collected by filtration. The precipitate was pushed and washed twice with 10 ml of water and twice with 10 ml of 60 v/v% aqueous solution of methanol and 10 ml of water, followed by vacuum drying at 60°C for 7 hours to obtain as a result 13.55 g of the desired product (yield 85.8).

As shown above, the production method of the present invention can produce the 2-acyliminopyridine derivative, which has extremely high yield and is represented by formula (I) above, in a short period of time.

On the other hand, it has been shown in the related art, for example Patent Literature 4 that the production using toluene as the solvent in the acylation step (corresponding to step 1 of the present invention) and acetonitrile as the solvent in the alkylation step (corresponding to step 2 of the present invention) has an overall yield of 83.9%, and the reaction time in step 2 is 18 hours.

Therefore, the superiority of the production method of the present invention over the related art was demonstrated by the following methods.

### (Example 14 and Comparative Examples 12 and 13)

In Example 14, reaction was performed by changing the reaction temperature and the reaction time of step 2 in Example 1 from 60°C and 3 hours to 70°C and 1.5 hours. In addition, in Comparative Examples 12 and 13, reaction was performed by changing the solvents of step 2 in Examples 1 and 14 from DMF to acetonitrile in accordance with the description of Patent Literature 4. These results (yields) are shown in Table 5 together with the reaction conditions in step 2.

**[Table 5]**

| | Comparative Example 12 | Comparative Example 13 | Example 1 | Example 14 |
|---|---|---|---|---|
| Reaction Temperature | 60°C | 70°C | 60°C | 70°C |
| Solvent | Acetonitrile (MeCN) | | N,N-dimethylformamide (DMF) | |
| Reaction Time | 3 Hours | 1.5 Hours | 3 Hours | 1.5 Hours |
| Yield | 74.8% | 72.5% | 94.7% | 93.8% |

The results of comparison under the same conditions except for the solvent in step 2 showed that the yield of the desired product obtained by the production method of the present invention was significantly higher than those by conventional production methods, making it possible to demonstrate the superiority of the present invention.

### (Example 15)

For the purpose of demonstrating that the present invention is a method suitable for industrial production, production was performed whose scale was increased to the order of kilogram from the production having the order of gram. Specifically, added were 6.64 kg of ethyl trifluoroacetate, 4.01 kg of 2-aminopyridine, and 10.6 L of DMF in this order, and after dissolution, 8.36 kg of sodium methoxide (28.0% methanol solution) was added dropwise thereto at room temperature. After stirring at 25°C for 1 hour, methanol and ethanol were distilled off under reduced pressure. A solution prepared by dissolving 7.06 kg of 2-chloro-5-chloromethyl pyridine in 1.68 L of DMF was added thereto, followed by stirring at 60°C for 3 hours. Thereafter, 5.84 kg of water and 7.36 L of methanol were added, and after stirring at room temperature overnight, the precipitate was collected by filtration. After pushing and washing with 19 L in total of 60 v/v% aqueous solution of methanol, vacuum drying at 70°C overnight was performed to obtain as a result 12.60 kg of the desired product (yield 93.8% and purity 98.3%). Therefore, it was also possible to demonstrate that the present invention is an industrially suitable production method.

### [Industrial Applicability]

The present invention makes it possible to produce in a one-pot manner a 2-acyliminopyridine derivative represented by formula (I) above, which is useful as a pest control agent, in an industrially advantageous manner as needed. In addition, the derivative can be produced without generating waste such as a pyridinate. Therefore, the present invention makes it possible to supply the above derivative in an amount required as a pest control agent with less burden on the environment, stably, and inexpensively.

## Claims

1. A method for producing a compound represented by the following formula (I) 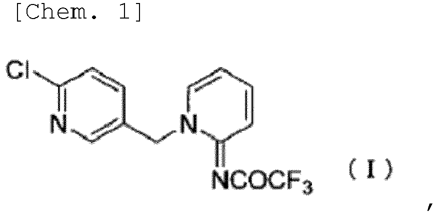 the method comprising, as shown in the following reaction formula: 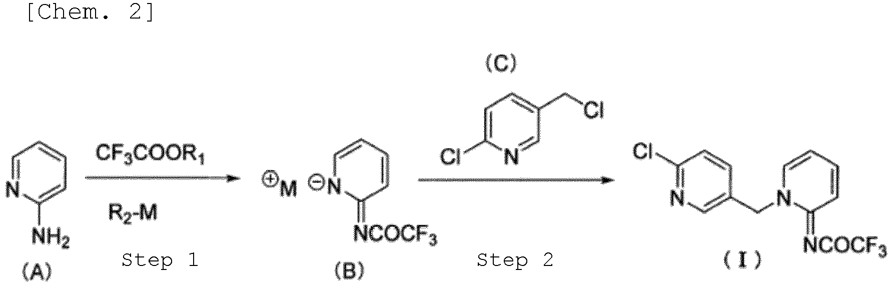 [where R₁ represents an ethyl group, R₂ represents a methyloxy group, and M represents a sodium atom],
step 1 of producing a compound represented by formula (B) by acylating an amino group at position 2 of a compound represented by formula (A) using a trifluoroacetic acid ester and a metal base; and
step 2 of alkylating a nitrogen atom at position 1 of the compound represented by formula (B) using a compound represented by formula (C), and wherein
the acylation reaction using the trifluoroacetic acid ester is performed in a solvent which consists of N,N-dimethylformamide and methanol.

2. The production method according to claim 1 wherein
the step 2 includes alkylating the nitrogen atom at position 1 of the compound represented by formula (B) using the compound represented by formula (C) in a solvent containing N,N-dimethylformamide.

3. The production method according to any one of claims 1 to 2, wherein
in the step 1, an amount of the metal base used is 0.95 to 1.1 equivalents relative to the compound represented by formula (A), and an amount of the trifluoroacetic acid ester used is 1.0 to 1.5 equivalents relative to the compound represented by formula (A).

4. A method for producing a compound represented by formula (I), comprising
producing the compound represented by formula (I) in a one-pot manner from a compound represented by formula (A) by adding a trifluoroacetic acid ester, a metal base, and a compound represented by formula (C) in the same reaction vessel as shown in the following reaction formula: 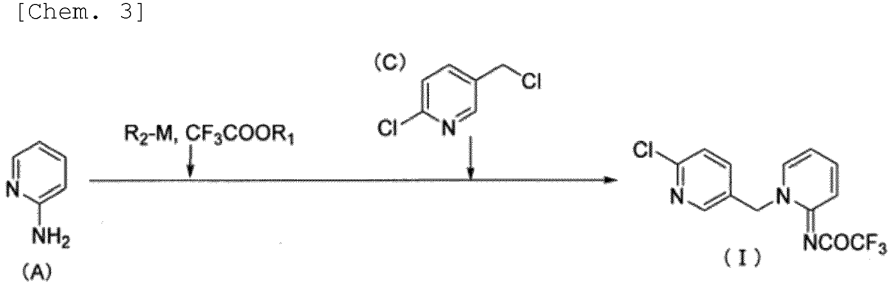
[where R₁ represents an ethyl group, R₂ represents a methyloxy group, and M represents a sodium atom], and wherein
an acylation reaction using the trifluoroacetic acid ester is performed in a solvent which consists of N,N-dimethylformamide and methanol.

5. The production method according to claim 4 wherein
an alkylation reaction using the compound represented by formula (C) is performed in a solvent containing N,N-dimethylformamide.

## Patentansprüche

1. Verfahren zur Herstellung einer Verbindung, die durch folgende Formel (I) dargestellt wird 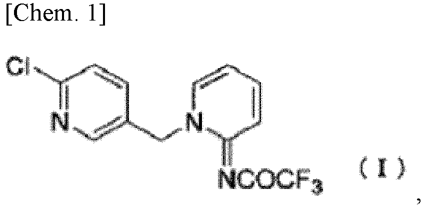 wobei das Verfahren, wie in der folgenden Reaktionsformel gezeigt, Folgendes umfasst: 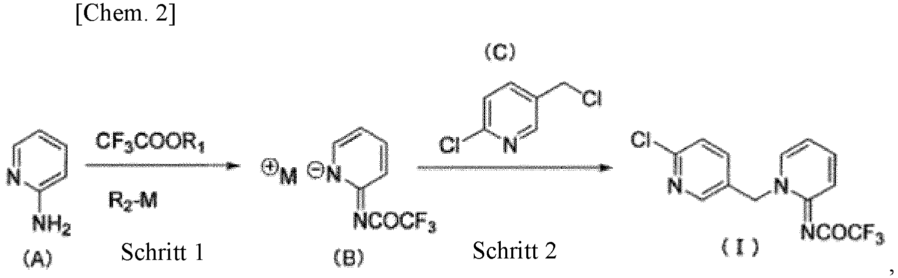
[wobei R₁ eine Ethylgruppe darstellt, R₂ eine Methyloxygruppe darstellt und M ein Natriumatom darstellt],
wobei Schritt 1 eine Verbindung der Formel (B) durch Acylierung einer Aminogruppe in Position 2 einer Verbindung der Formel (A) unter Verwendung eines Trifluoressigsäureesters und einer Metallbase herstellt; und
Schritt 2 eine Alkylierung eines Stickstoffatoms in Position 1 der durch Formel (B) dargestellten Verbindung unter Verwendung einer durch Formel (C) dargestellten Verbindung bewirkt, und wobei
die Acylierungsreaktion unter Verwendung des Trifluoressigsäureesters in einem Lösungsmittel durchgeführt wird, das aus N,N-Dimethylformamid und Methanol besteht.

2. Herstellungsverfahren nach Anspruch 1, wobei
Schritt 2 die Alkylierung des Stickstoffatoms in Position 1 der durch Formel (B) dargestellten Verbindung unter Verwendung der durch Formel (C) dargestellten Verbindung in einem N,N-Dimethylformamid enthaltenden Lösungsmittel umfasst.

3. Herstellungsverfahren nach einem der Ansprüche 1 bis 2, wobei
in Schritt 1 die Menge der verwendeten Metallbase 0,95 bis 1,1 Äquivalente, bezogen auf die durch Formel (A) dargestellte Verbindung, beträgt, und eine Menge des verwendeten Trifluoressigsäureesters 1,0 bis 1,5 Äquivalente, bezogen auf die durch Formel (A) dargestellte Verbindung, beträgt.

4. Verfahren zur Herstellung einer durch Formel (I) dargestellten Verbindung, das Folgendes umfasst:
Herstellen der durch Formel (I) dargestellten Verbindung in einer Eintopfmethode aus einer durch Formel (A) dargestellten Verbindung durch Zugabe eines Trifluoressigsäureesters, einer Metallbase und einer Verbindung der Formel (C) in demselben Reaktionsgefäß, wie in der folgenden Reaktionsformel gezeigt: 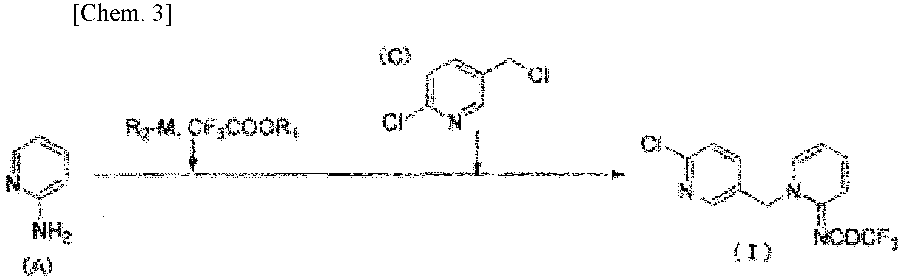
[wobei R₁ eine Ethylgruppe darstellt, R₂ eine Methyloxygruppe darstellt und M ein Natriumatom darstellt], und wobei
eine Acylierungsreaktion unter Verwendung des Trifluoressigsäureesters in einem Lösungsmittel durchgeführt wird, das aus N,N-Dimethylformamid und Methanol besteht.

5. Herstellungsverfahren nach Anspruch 4, wobei eine Alkylierungsreaktion unter Verwendung der durch Formel (C) dargestellten Verbindung in einem N,N-Dimethylformamid enthaltenden Lösungsmittel durchgeführt wird.

## Revendications

1. Procédé de production d'un composé représenté par la formule (I) suivante 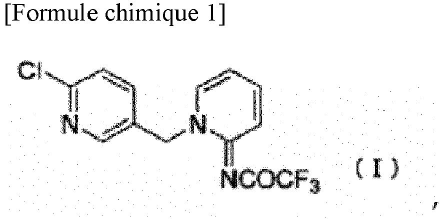 le procédé comprenant, comme indiqué dans la formule réactionnelle suivante : 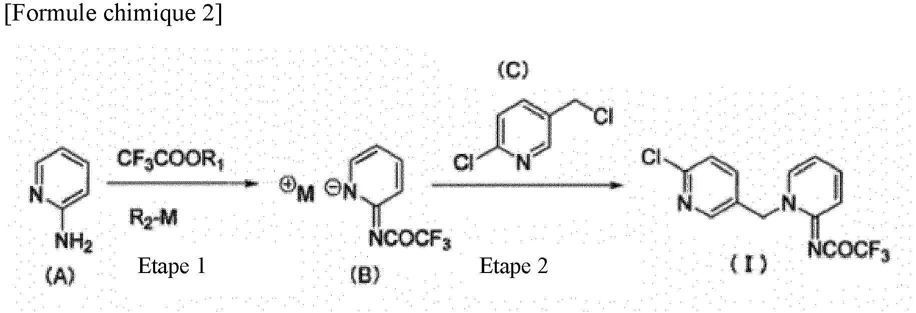
[où R₁ représente un groupe éthyle, R₂ représente un groupe méthyloxy et M représente un atome de sodium],
l'étape 1 de production d'un composé représenté par la formule (B) en acylant un groupe amino en position 2 d'un composé représenté par la formule (A) en utilisant un ester d'acide trifluoroacétique et une base métallique ; et
l'étape 2 d'alkylation d'un atome d'azote en position 1 du composé représenté par la formule (B) en utilisant un composé représenté par la formule (C), et dans lequel
la réaction d'acylation utilisant l'ester d'acide trifluoroacétique est effectuée dans un solvant qui est constitué de N, N-diméthylformamide et de méthanol.

2. Procédé de production selon la revendication 1, dans lequel
l'étape 2 comprend l'alkylation de l'atome d'azote en position 1 du composé représenté par la formule (B) en utilisant le composé représenté par la formule (C) dans un solvant contenant du N,N-diméthylformamide.

3. Procédé de fabrication selon l'une quelconque des revendications 1 à 2, dans lequel à l'étape 1, une quantité de la base métallique utilisée est de 0,95 à 1,1 équivalents par rapport au composé représenté par la formule (A), et une quantité de la base d'ester d'acide trifluoroacétique utilisée est de 1,0 à 1,5 équivalents par rapport au composé représenté par la formule (A).

4. Procédé de production d'un composé représenté par la formule (I), comprenant la production du composé représenté par la formule (I) en un récipient unique à partir d'un composé représenté par la formule (A) en ajoutant un ester d'acide trifluoroacétique, une base métallique, et un composé représenté par la formule (C) dans le même récipient de réaction comme indiqué dans la formule de réaction suivante : 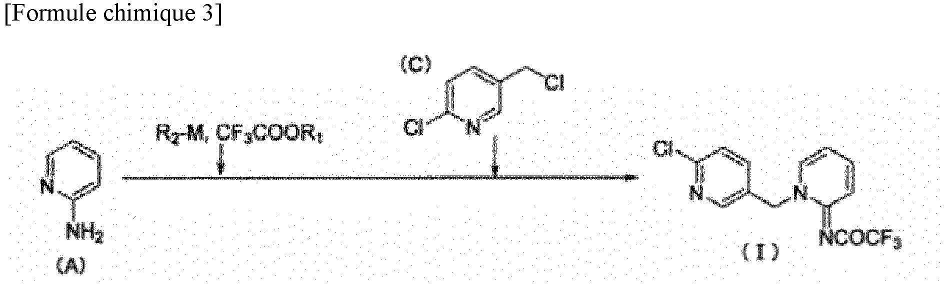
[où R₁ représente un groupe éthyle, R₂ représente un groupe méthyloxy et M représente un atome de sodium], et dans lequel
une réaction d'acylation utilisant l'ester d'acide trifluoroacétique est effectuée dans un solvant constitué de N,N-diméthylformamide et de méthanol.

5. Procédé de production selon la revendication 4, dans lequel
une réaction d'alkylation utilisant le composé représenté par la formule (C) est effectuée dans un solvant contenant du N,N-diméthylformamide.
